# EUROPEAN PATENT SPECIFICATION

(11) **EP 0 325 616 B1**
(45) Date of publication and mention of the grant of the patent: **20.07.1994**
(21) Application number: 87907073.8
(22) Date of filing: 07.10.1987
(51) Int. Cl.: A61K 31/445, A61K 31/55, A61K 31/33, A61K 31/70, A61K 31/56, A61K 31/505, A61K 31/34, A61K 31/04, A61K 31/415, A61K 31/075

(54) **PENETRATION ENHANCERS FOR TRANSDERMAL DELIVERY OF SYSTEMIC AGENTS**
DURCHDRINGUNGSERHÖHENDE MITTEL FÜR TRANSDERMALE VERABREICHUNG SYSTEMISCHER MITTEL
ADJUVANTS DE PENETRATION POUR L'ADMINISTRATION TRANSDERMIQUE D'AGENTS SYSTEMIQUES

(30) Priority: 07.10.1986 US 916402
(43) Date of publication of application: 02.08.1989
(73) Proprietor: WHITBY RESEARCH, Inc., Richmond, VA 23227 (US)
(72) Inventor: MINASKANIAN, Gevork, Irvine, CA 92714 (US); PECK, James, V., Costa Mesa, CA 92626 (US)
(74) Representative: KOHLER SCHMID + PARTNER
(86) International application number: US8702582
(87) International publication number: WO8802626

(56) References cited:
- EP-B- 129 284
- EP-B- 0 029 618
- WO-A-87/04706
- FR-B-02 519 549
- JP-B- 6 133 128
- JP-B-61 129 140
- US-A- 3 157 641
- US-A- 3 306 910
- US-A- 3 306 911
- US-A- 3 823 152
- US-A- 3 865 814
- US-A- 3 989 815
- US-A- 3 989 816
- US-A- 3 991 203
- US-A- 4 122 170
- US-A- 4 310 525
- US-A- 4 311 481
- US-A- 4 316 893
- US-A- 4 405 616
- US-A- 4 415 563
- US-A- 4 423 040
- US-A- 4 424 210
- US-A- 4 444 762
- US-A- 4 525 199
- US-A- 4 537 776
- US-A- 4 552 872
- US-A- 4 557 934
- US-A- 4 562 075
- US-A- 4 605 670
- US-A- 4 620 949
- US-A- 4 626 539
- US-A- 4 637 930
- Chemical Abstracts, vol. 99, Abstract no. 76694u (STOUGHTON ET AL.), "Azone.a new non-toxic enhancer of cutaneous penetration" (1983)
- Chemical Abstracts, vol. 97 Abstract no. 78799q (STOUGHTON) "Enhanced percutaneous penetration with 1-dodecylazacycloheptan- -2-one" (1982)
- Chemical Abstracts, vol. 103, Abstractno. 147082q (SUGIBAYASHI ET AL.) "Effect of the absorption enhancer, azone, on the transport of 5-fluoro-uracil across hairless rat skin" (1985)
- CHEMICAL Abstracts,vol. 103, Abstract no. 27169u (WOTTON ET AL) "Vehicle effect on topical drug delivery. Effect of azone on the cutaneous permeation of metronidazole and propylene glycol" (1985)
- Chemical Abstracts vol. 105, Abstract no. 29927e RYATT ET AL. "Pharmacodynamic measurement of percutaneous penetration enhancement in vivo" (1986)
- Chemical Abstract vol. 104, Abstract no. 28446q SHANNON ET AL.) "Influence of 1-dodecylazacycloheptan-2-one (azone).... 5 -O-valery-9- -D-arabinofuranosyladenine" (1985)
- Chemical Abstracts vol. 104, Abstract no. 10508b (HADGRAFT ET AL.) "Facilitated transport of sodium salicylate across an artificial lipid membrane by Azone" (1985)
- Chemical Abstracts, vol. 104, Abstract no. 115982e (HOELGAARD ET AL.) "Dermal drug delivery-improvement by choice of vehicle or drug derivative". (1985)

## Description

### 1) Field of the Invention

The invention generally relates to an improved method of drug delivery. More particularly, the invention relates to the use of an improved membrane penetration enhancer in a medicament for enhancing the transdermal delivery of certain systemically active drugs to humans and animals.

### 2) Background of the Prior Art

For some years, pharmaceutical researchers have sought an effective means of introducing drugs into the bloodstream by applying them to unbroken skin. Among other advantages, such administration can provide a comfortable, convenient, and safe way of giving many drugs now taken orally or infused into veins or injected intramuscularly.

Using skin as the portal for drug entry offers unique potential, because transdermal delivery permits close control over drug absorption. For example, it avoids factors that can cause unpredictable absorption from the gastrointestinal tract, including: changes in acidity, motility, and food content. It also avoids initial metabolism of the drug by the liver. Thus, controlled drug entry through skin can achieve a high degree of control over blood concentrations of drug.

Close control over drug concentrations in blood can translate readily into safer and more comfortable treatment. When a drug's adverse effects occur at higher concentrations than its beneficial ones, rate control can maintain the concentrations that evoke only--or principally the drug's desired actions. This ability to lessen undesired drug actions can greatly reduce the toxicity hazards that now restrict or prevent the use of many valuable agents.

Transdermal delivery particularly benefits patients with chronic disease. Many such patients have difficulty following regimens requiring several doses daily of medications that repeatedly cause unpleasant symptoms. They find the same drugs much more acceptable when administered in transdermal systems that require application infrequently--in some cases, only once or twice weekly--and that reduce adverse effects.

Transdermal delivery is feasible for drugs effective in amounts that can pass through the skin area and that are substantially free of localized irritating or allergic effects. While these limitations may exclude some agents, many others remain eligible for transdermal delivery. Moreover, their numbers will expand as pharmaceutical agents of greater potency are developed. Particularly suitable for transdermal delivery are potent drugs with only a narrow spread between their toxic and safe blood concentrations, those having gastrointestinal absorption problems, or those requiring frequent dosing in oral or injectable form.

Transdermal therapy permits much wider use of natural substances such as hormones. Often the survival times of these substances in the body are so short that they would have to be taken many times daily in ordinary dosage forms. Continuous transdermal delivery provides a practical way of giving them, and one that can mimic the body's own patterns of secretion.

At present, controlled transdermal therapy appears feasible for many drugs used for a wide variety of ailments including, but not limited to, circulatory problems, hormone deficiency, respiratory ailments, and pain relief.

Percutaneous administration can have the advantage of permitting continuous administration of drug to the circulation over a prolonged period of time to obtain a uniform delivery rate and blood level of drug. Commencement and termination of drug therapy are initiated by the application and removal of the dosing devices from the skin. Uncertainties of administration through the gastrointestinal tract and the inconvenience of administration by injection are eliminated. Since a high concentration of drug never enters the body, problems of pulse entry are overcome and metabolic half-life is not a factor of controlling importance.

U.S. Patent Nos. 3,989,815; 3,989,816; 3,991,203; 4,122,170; 4,316,893; 4,415,563; 4,423,040; 4,424,210; and 4,444,762 generally describe a method for enhancing the topical (as contrasted to the systemic) administration of physiologically active agents by combining such an agent with an effective amount of a penetration enhancer and applying the combination topically to humans or animals, in the form of creams, lotions, gels.

Penetration enhancers for enhancing systemic administration of therapeutic agents transdermally are cited in U.S. Patent Nos. 4,405,616; 4,562,075; 4,031,894, 3,996,934; and 3,921,636.

Further, in WO-A 87 04 706 which forms a prior art reference under Art. 54 (3) compositions are described which comprise a penetration enhancer as indicated in the present application together with certain active agents.

EP-A 0 129 284 and US-A 4 405 616 describe the use of penetration enhancers for the topical delivery of systemic active agents. The pentration enhancer used differs from those of the present application in that in the structural formula the substituent x represents oxygen.

### SUMMARY OF THE INVENTION

It has been discovered that the penetration enhancers previously disclosed in the patents mentioned above to enhance topical delivery of physiologically active agents also enhance the transdermal delivery of systemically active agents through the skin or other body membranes of humans and animals directly into the bloodstream.

The invention therefore provides the use of a compound selected from the group of compounds represented by the structural formula 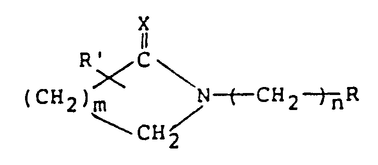
wherein X represents sulfur or two hydrogen atoms; R' is H or a lower alkyl group having 1-4 carbon atoms; m is 2-6; n is 0-18 and R is -CH₃, 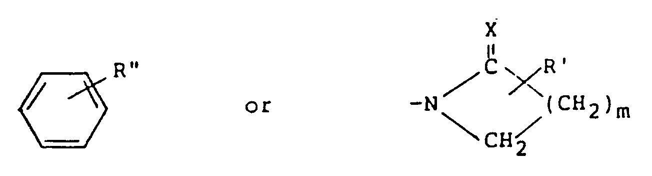
wherein R'' is H or halogen for the manufacture of a medicament for enhancing the transdermal delivery of systemically active agents selected from the group consisting of haloperidol, isosorbide dinitrate, nitroglycerin, estradiol, clonidine, dichlorofenac and metaproterenol through the skin or other body membranes of humans and animals.

Preferably R is -CH₃ and R' is H.

In a more preferred embodiment of the present invention R is -CH₃, R' is H and m equals 4. Even more preferably n is 4-17, e.g. 11.

### DETAILED DESCRIPTION OF THE INVENTION

Typical examples of compounds represented by the above structural formula include:
1-n-Dodecylazacycloheptane
1-n-Dodecylazacycloheptan-2-thione
1-n-Butylazacyclohexan-2-thione
1-n-Butylazacyclopentan-2-thione
1-n-Octylazacyclopentan-2-thione
1-n-Dodecylazacyclopentan-2-thione
1-n-Butylazacycloheptan-2-thione
1-n-Octylazacycloheptan-2-thione
1,1'-Hexamethylenediazacyclopentan-2-thione
1-n-Dodecylazacyclohexan-2-thione
1-n-Decylazacycloheptane
1-n-Hexadecylazacycloheptane
1-n-Octadecylazacycloheptane
1-n-Undecylazacycloheptane
1-n-Tetradecylazacycloheptane
Typical systemically active agents which are delivered transdermally are those mentioned above.

Dosage forms for application to the skin or other membranes of humans and animals include creams, lotions, gels, ointments, suppositories, sprays, aerosols, buccal and sub-lingual tablets and any one of a varietary of transdermal devices for use in the continuous administration of systemically active drugs by absorption through the skin, oral mucosa or other membranes, see, for example, one or more of U.S. Patents Nos. 3,598,122; 3,598,123; 3,731,683; 3,742,951; 3,814,097; 3,921,636; 3,972,995; 3,993,072; 3,993,073, 3,996,934; 4,031,894; 4,060,084; 4,069,307; 4,201,211; 4,230,105; 4,292,299 and 4,292,303.

Typical inert carriers which may be included in the foregoing dosage forms include conventional formulating materials, such as, for example, water, isopropyl alcohol, gaseous fluorocarbons, ethyl alcohol, polyvinyl pyrrolidone, propylene glycol, fragrances, gel-producing materials such as "Carbopol®", stearyl alcohol, stearic acid, spermaceti, sorbitan monooleate, "Polysorbates®", "Tweens®", sorbital, methylcellulose.

Systemically active agents are used in amounts calculated to achieve and maintain therapeutic blood levels in a human or animal over the period of time desired. These amounts vary with the potency of each systemically active substance, the amount required for the desired therapeutic or other effect, the rate of elimination or breakdown of the substance by the body once it has entered the bloodstream and the amount of penetration-enhancer in the formulation. In accordance with conventional prudent formulating practices, a dosage near the lower end of the useful range of a particular agent is usually employed initially and the dosage increased or decreased as indicated from the observed response, as in the routine procedure of the physician.

The amount of penetration enhancer which may be used in the invention varies from 1 to 100 percent although adequate enhancement of penetration is generally found to occur in the range of 1 to 10 percent by weight of the formulation to be delivered. The penetration-enhancer disclosed herein may be used in combination with the active agent or may be used separately as a pre-treatment of the skin or other body membrane through which the systemically-active agent is intended to be delivered.

The invention is further illustrated by the following examples which are illustrative of a specific mode of practicing the invention

### EXAMPLE 1

A composition, in the form of a gel, suitable for transdermal delivery of haloperidol, an antidyskinetic or antipsychotic drug, is prepared by mixing the following components in the given concentrations.

| Component | Weight % |
|---|---|
| Haloperidol | 1-5 |
| Penetration enhancer | 1-10 |
| Carbopol® 934 P (Available from B.F. Goodrich) | 0.5-2 |
| Neutralizing Agent (NaOH) | q.s. |
| Tween®-20 (Available from Atlas Chemical, a Div. of I.C.I.) | 1-10 |
| Preservative (Sorbic Acid) | q.s. |
| Antioxidant (Ascorbic Acid) | q.s. |
| Chelating Agent (Disodium salt of ethylenediaminetetra acetic acid) | q.s. |
| Deionized Water | q.s. to 100 |

This composition is topically applied to the skin of a human subject and after the passage of a suitable period of time haloperidol is found in the bloodstream of said subject.

### Example 2

When an amine, e.g. triethylamine or triethanolamine, is substituted for NaOH the results are substantially similar, i.e. a topical composition suitable for transdermally delivering haloperidol to the bloodstream is obtained.

### Example 3

When potassium sorbate, or a lower alkyl paraben, e.g. methyl, ethyl, propyl or butyl paraben are substituted for the preservative of the composition of Example 1, the results are substantially similar, i.e. a topical composition suitable for the transdermal delivery of haloperidol to the bloodstream is obtained.

### Example 4

When ascorbyl palmitate, Vitamin E, thioglycerol, thioglycolic acid, sodium formaldehyde sulfoxylate, BHA, BHT, propyl gallate or sodium metabisulfite are substituted for the antioxidant of the composition formulated in Example 1, the results are substantially similar in that a topical composition suitable for transdermally delivering haloperidol to the bloodstream is obtained.

### Example 5

The composition of Example 1 is prepared in the form of a sodium alginate gel by mixing the following components in the following given concentrations:

| Component | Weight % |
|---|---|
| Haloperidol | 1-5 |
| Penetration enhancer | 1-10 |
| Sodium Alginate | 0.5-5 |
| Calcium Salts | q.s. |
| Tween®-20 | 1-10 |
| Preservative* | q.s. |
| Antioxidant** | q.s. |
| Chelating Agent*** | q.s. |
| Deionized Water | to 100 |

| | |
|---|---|
| *Suitable preservatives are those used in Example 3 as well as sorbic acid. | |
| **Suitable antioxidants are those used in Example 4 including ascorbic acid. | |
| ***The chelating agent is the disodium salt of ethylenediaminetetraacetic acid. | |

This composition when applied topically is found to transdermally deliver haloperidol to the bloodstream of a subject.

### Example 6

The composition of Example 1 is prepared in the form of a hydrophilic cream by mixing the following components.

| Component | Weight % |
|---|---|
| Oil Phase | |
| Cetyl Alcohol | 5-15 |
| Stearyl Alcohol | 1-5 |
| Penetration enhancer | 0.5-10 |
| Glycerol Monostearate | 2-7 |

| Water Phase | |
|---|---|
| Sodium Laurylsulfate | 0.1 |
| Solvent* | 2-20 |
| Tween®-20 | 1-5 |
| Water | q.s. to 100 |

| | |
|---|---|
| *Suitable solvents are propylene glycol, glycerin, alcohols, for example, ethyl alcohol, isopropylalcohol, etc. and polyethylene glycols. | |

The oil phase and the water phase is made up separately, and then agitated to form an emulsion. (When, as in Example 8, the active ingredient, is other than haloperidol, depending on its lipophilicity, it will be distributed in the oil or water phase.) This hydrophilic cream, when applied topically to the skin of a human, is found to transdermally delivery haloperidol into the bloodstream.

### Example 7

The composition of the instant invention may also be delivered by use of a polymeric matrix. For example, a solid polymer such as cellulose triacetate, polyvinyl acetate, terpolymers and copolymers of vinyl chloride and vinyl acetate, copolymers of polyvinyl alcohol and polyvinyl acetate, and silicon elastomers is imbibed with a liquid having the following components in the given concentrations.

| Component | Weight % |
|---|---|
| Polymer | 5-40 |
| Haloperidol | q.s. |
| Penetration enhancer | 0.5-80 |
| Solvent* | 5-90 |
| Surfactant** | 1-10 |
| Preservative*** | q.s. |
| Antioxidant**** | q.s. |

| | |
|---|---|
| *Solvents may be the solvents used in Example 6 above. | |
| **The Surfactant may be Tween®-20, glycerolmonostearate or sodium laurylsulfate, etc. | |
| ***The preservative may be any of the preservatives used in Example 3 above. | |
| ****The antioxidants may be any of those used in Example 4 above. | |

When solid matrix, containing the active ingredients formulated above, is contacted with the skin of a human subject, after a period of time the active agent is found in the bloodstream of said subject.

### Example 8

Examples 1 to 7 are repeated except that the following active ingredients in the given concentrations are substituted for haloperidol:

| Active Ingredient | Weight % |
|---|---|
| Isosorbide Dinitrate | 5-15 |
| Nitroglycerin | 1-5 |
| Estradiol | 1-5 |
| Clonidine | 0.5-3 |
| Propranolol | 1-5 |
| Nifedipine | 1-5 |
| Nicardipine | 1-5 |
| Dichlorofenac | 5-15 |
| Metaproterenol | 1-5 |

Similar results are obtained in that the active ingredient is transdermally delivered to the bloodstream of an animal.

## Claims

1. The use of a compound selected from the group of compounds represented by the structural formula 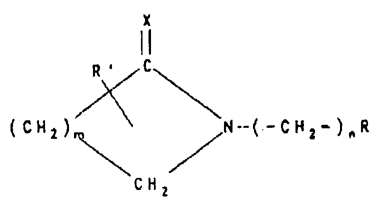 wherein X represents sulfur or two hydrogen atoms; R' is H or a lower alkyl group having 1 to 4 carbon atoms; m is 2 to 6; n is 0 to 18 and R is - CH₃ 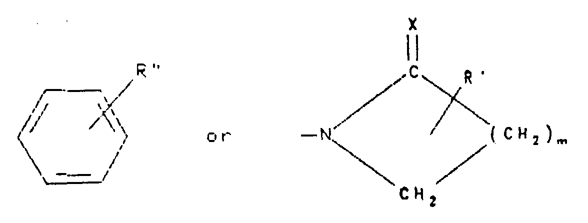 wherein R'' is H or halogen, for the manufacture of a medicament for enhancing the transdermal delivery of systemically active agents selected from haloperidol, isosorbide dinitrate, nitroglycerin, estradiol, clonidine, dichlorofenac, propranolol, nifedipine, nicardipine, and metaproterenol, through the skin or other body membranes of humans and animals.

2. The use of claim 1 wherein R' is hydrogen, m is 4, n is 4 to 17 and R is -CH₃.

3. The use of claim 2 wherein X is sulfur.

4. The use of claim 2 wherein X represents two hydrogen atoms.

5. The use of claim 3 wherein n is 11.

6. The use of claim 4 wherein n is 11.

7. The use of claim 1 wherein the administration is concurrent.

## Patentansprüche

1. Verwendung einer Verbindung ausgewählt aus der Gruppe von Verbindungen mit der Strukturformel 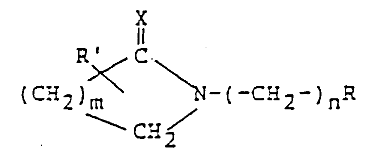 in der X Schwefel oder zwei Wasserstoffatome bedeutet; R' H oder eine Niedrigalkylgruppe mit 1 bis 4 Kohlensstoffatomen ist, m 2 bis 6 ist; n 0 bis 18 und R -CH₃ ist, 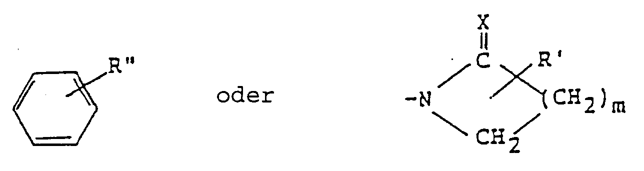 in der R'' H oder Halogen ist, zur Herstellung eines Medikaments zur Verbesserung der transdermalen Verabreichung von systemisch aktiven Mitteln, die aus Haloperidol, Isosorbiddinitrat, Nitroglycerin, Estradiol, Clonidin, Dichlorfenac, Propranolol, Nifedipin, Nicardipin und Mecaproterenol ausgewählt sind, durch die Haut oder andere Körpermembranen von Menschen und Tieren.

2. Verwendung nach Anspruch 1, bei der R' Wasserstoff, m 4, n 4 bis 17 und R -CH₃ ist.

3. Verwendung nach Anspruch 2, bei der X Schwefel ist.

4. Verwendung nach Anspruch 2, bei der X zwei Wasserstoffatome bedeutet.

5. Verwendung nach Anspruch 3, bei der n 11 ist.

6. Verwendung nach Anspruch 4, bei der n 11 ist.

7. Verwendung nach Anspruch 1, bei der die Verabreichung gleichzeitig erfolgt.

## Revendications

1. Utilisation d'un composé choisi dans le groupe des composés représentés par la structure de formule 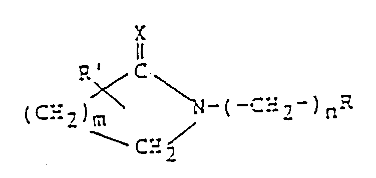 dans laquelle X représente le soufre ou deux atomes d'hydrogène; R' es H ou un groupe alkyle inférieur possédant 1 à 4 atomes de carbone; m équivaut de 2 à 6; n équivaut de 0 à 18 et R est -CH₃ 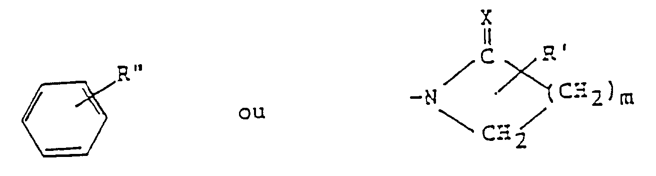 dans laquelle R'' est H ou un halogène, pour la fabrication d'un médicament pour augmenter la libération à travers le derme de principes systématiquement actifs choisis parmi l'halopéridole, le dinitrate d'isosorbide, la nitroglycérine, l'oestradiol, la clonidine, le dichlorofénac, le propranolol , la nifédipine, la nicardipine , et le métaprotérénol, à travers la peau ou d'autres membranes chez l'homme ou l'animal.

2. Utilisation selon la revendication 1 dans laquelle R' est l'hydrogène, m est égal à 4, n équivaut de 4 à 17 et R est -CH₃.

3. Utilisation selon la revendication 2 dans laquelle X est le soufre.

4. Utilisation selon la revendication 2 dans laquelle X représente deux atomes d'hydrogène.

5. Utilisation selon la revendication 3 dans laquelle n est égal à 11.

6. Utilisation selon la revendication 4 dans laquelle n est égal à 11.

7. Utilisation selon la revendication 1 dans laquelle l'administration est concourante.
